# EUROPEAN PATENT APPLICATION

(11) **EP 4 437 836 A1**
(43) Date of publication of application: **02.10.2024**
(21) Application number: 22898542.0
(22) Date of filing: 21.11.2022
(51) Int. Cl.: A01G 7/00, A01G 22/05, A01G 24/10, A01G 24/20

(54) **CULTURE SOIL FOR PLANT, CULTIVATION SET INCLUDING CULTURE SOIL, CULTIVATION METHOD USING CULTURE SOIL, AND SEEDLING OF PLANT WITH CULTURE SOIL**

(30) Priority: 24.11.2021 JP 2021190595
(71) Applicant: Ibaraki University, Ibaraki 310-8512 (JP)
(72) Inventor: NARISAWA, Kazuhiko, Inashiki-gun Ibaraki 300-0393 (JP); WIWIEK, Harsonowati, Inashiki-gun Ibaraki 300-0393 (JP)
(74) Representative: Berggren Oy
(86) International application number: PCT/JP2022/043068
(87) International publication number: WO 2023/095759

(57) **Abstract**

A culture soil for plants includes a fungus of genus *Cladophialophora,* and one kind of soil of strongly acidic organic cultivation soil, strongly acidic conventional cultivation soil, or weakly acidic to neutral organic cultivation soil. Alternatively, a culture soil for plants contains fungi, which are *Exophiala* sp. SK47 or a mutant isolate thereof, and strongly acidic organic cultivation soil or strongly acidic conventional cultivation soil; a method of cultivating plant using the culture soil; and a seedling of a plant with culture soil are also provided.

## Description

### Technical Field

The present disclosure relates to a culture soil for plants, a cultivation set including culture soil, a cultivation method using culture soil, and a seedling of a plant with culture soil.

### Background Art

According to the Food and Agricultural Organization of the United Nations, the agricultural land area where crops can be produced without problems is about 40% of the total agricultural land area in the world. Currently, about 5 million ha of agricultural land is further deteriorated per year, and unless new efforts are made to secure the agricultural land, it is expected that in 2050, the arable land per person in the world will decrease to 1/4 of the level of the arable land in 1960. For this reason, it is necessary to convert conventional agriculture that places importance on productivity to sustainable agriculture that places importance on the environment.

In conventional agriculture that emphasize productivity, there is a case where only a specific type of crop is cultivated in a specific agricultural land. However, by continuing to cultivate the same crop in the same place, the growth of certain types of pathogens (for example, pests or pathogenic microorganisms, etc) is promoted, the balance of the ecosystem is lost, and the production of the crop is damaged. This is because many of the pathogens have host specificity for the crop. Therefore, as a method of solving this problem, a cultivation method using dark-septate endophytes is known. Dark-septate endophytes (DSE) are useful fungi living in plant roots and having a symbiotic relationship with plants.

As a cultivation method using a useful fungi having a symbiotic relationship with a plant, for example, the cultivation method is reported in which *Cladophialophora chaetospira* SK 51, which is a fungus, is allowed to coexist in a strawberry seedling in which chlorosis has been induced by a fungus belonging to the genus *Fusarium* (For example, Wiwiek Harsonowati et al., The Effectiveness of a Dark Septate Endophytic Fungus, Cladophialophora chaetospira SK 51, to Mitigate Strawberry Fusarium Wilt Disease and With Growth Promotion Activities, Front. Microbiol., 2020. Apr. 15, Vol. 11, Art. 585).

### SUMMARY OF INVENTION

### Problem to be Solved by Invention

However, at present, conventional agriculture that places importance on productivity is the mainstream, and knowledge for sustainable agriculture that places importance on the environment is limited.

The present disclosure has been made in view of the above, and an object of the disclosure is to provide a culture soil for plants capable of promoting plant growth or flower bud formation under conditions usually not suitable for plant growth, a cultivation set including the culture soil, a cultivation method using the culture soil, and a seedling of a plant with culture soil.

### Means for Solving the Problem

Specific means for solving the problems include the following aspects.
<1> A culture soil for plants, including:
   a fungus of genus *Cladophialophora*; and
   any one kind of soil of strongly acidic organic cultivation soil, strongly acidic conventional cultivation soil, or weakly acidic to neutral organic cultivation soil.
<2> The culture soil for plants according to <1>, in which
   the fungus of the genus *Cladophialophora* is *Cladophialophora chaetospira.*
<3> Culture soil for plants, including:
   a fungus of *Exophiala* sp. SK 47 or a mutant isolate thereof; and
   strongly acidic organic cultivation soil or strongly acidic conventional cultivation soil.
<4> The culture soil for plants according to any one of <1> to <3>, in which
   the strong acidity is pH 3 or more and less than pH 5.
<5> The culture soil for plants according to any one of <1> to <4>, in which
   the plant is a fruit plant or a vegetable plant.
<6> A cultivation set including:
   the culture soil for plants according to any one of <1> to <5>; and
   a plant body.
<7> A method of cultivating a plant, including:
   a cultivation step of cultivating a plant using the culture soil for plants according to any one of <1> to <5>.
<8> The method of cultivating a plant according to <7>, in which
   in the cultivation step, a plant in which flower bud formation occurs under a low-temperature and short-day condition is cultivated under a high-temperature and long-day condition.
<9> The method of cultivating a plant according to <8>, in which
   the high-temperature and long-day condition includes a temperature in a range of more than 15°C and 25°C or less, and a day length in a range of more than 12 hours and 24 hours or less.
<10> A seedling of a plant with culture soil, including:
   the culture soil for plants according to any one of <1> to <5>; and
   a seedling of a plant.

### Advantageous Effects of Invention

According to the present disclosure, there are provided culture soil for plants which is capable of promoting plant growth or flower bud formation under conditions that are usually not suitable for plant growth, a cultivation set including the culture soil, a cultivation method using the culture soil, and a seedling of a plant.

### BRIEF DESCRIPTION OF DRAWINGS

Fig. 1 is a photograph showing a growth state of strawberry.
Fig. 2 is a graph showing dry mass of an above-ground part of strawberry.
Fig. 3 is a graph showing dry mass of roots of strawberry.
Fig. 4 is a graph showing the number of fruits formed in strawberry.
Fig. 5 is a graph showing the number of days until flower bud formation in strawberry.

### DESCRIPTION OF EMBODIMENTS

Hereinafter, an embodiment of the present disclosure will be described in detail. However, the disclosure is not limited to the following embodiments. In the following disclosure, the constituent elements (including element steps and the like) are not essential unless otherwise specified. The same applies to numerical values and ranges thereof, and the disclosure is not limited thereto.

In the disclosure, the term "step" includes not only a step independent of other steps but also a step that cannot be clearly distinguished from other steps as long as the purpose of the step is achieved.

In the disclosure, a numerical range indicated using "to" includes numerical values described before and after "to" as a lower limit value and an upper limit value, respectively.

In the numerical ranges described in stages in the disclosure, the upper limit value or the lower limit value described in one numerical range may be replaced with the upper limit value or the lower limit value of the numerical range described in another stage. In addition, in the numerical range described in the present text, the upper limit value or the lower limit value of the numerical range may be replaced with a value shown in Examples.

In the disclosure, the content of each component in the composition means the total content of a plurality of substances corresponding to each component in the composition when the plurality of substances exists in the composition, unless otherwise specified.

### << Culture Soil for Plants of First Embodiment»

Culture soil for plants according to a first embodiment of the present disclosure includes a fungus of genus *Cladophialophora,* and any one kind of soil of strongly acidic organic cultivation soil, strongly acidic conventional cultivation soil, or weakly acidic to neutral organic cultivation soil.

The culture soil containing the fungus of the genus *Cladophialophora,* and any one of strongly acidic organic cultivation soil, strongly acidic conventional cultivation soil, and weakly acidic to neutral organic cultivation soil is culture soil capable of promoting plant growth or flower bud formation under conditions usually not suitable for plant growth.

The action of the culture soil for plants of the first embodiment is not clear, but is estimated as follows.

The fungus of the genus *Cladophialophora* contained in culture soil extends hyphae in a network shape around a root of plant. Then, when the fungus adheres to the surface of the root of the plant, it forms an appressorium, enters the cell interior of the root of the plant and settles. That is, a symbiosis between the fungus and the plant is established.

When the symbiosis is established, for example, when the culture soil is organic cultivation soil, fungi exist in the organic cultivation soil, and it is possible to provide plants with amino acids, proteins, or the like that cannot be efficiently used as nutrients for plants as they are. As a result, the plant easily absorbs nutrients such as nitrogen and phosphorus, and uses the nutrients for its own growth. Furthermore, for example, in a case where the culture soil is strongly acidic soil or in a case where plants are cultivated under conditions where the temperature or the day length is not suitable for the plants, fungi impart tolerance to these environmental stresses to the plants.

That is, even under conditions such as organic cultivation soil, strongly acidic soil, or temperature or day length not suitable for plant growth or flower bud formation, plant growth or flower bud formation is promoted by cultivating a plant using the culture soil for plants of the first embodiment.

Note that the disclosure is not limited to the estimation mechanism at all.

In the disclosure, it can be confirmed that the growth of the plant is promoted by increasing the dry mass of the above-ground part or increasing the dry mass of the root.

In the disclosure, promotion of flower bud formation of a plant can be confirmed by an increase in the number of flower buds to be formed or a decrease in the number of days until flower bud formation. Alternatively, in the disclosure, promotion of flower bud formation of the plant can be confirmed by an increase in the number of fruits to be formed or a decrease in the number of days until fruit formation. In the disclosure, the fruit does not refer to only true fruits, and includes false fruits such as strawberry and fig, for example.

### <Fungi>

### (Fungi of the Genus Cladophialophora)

Examples of the fungus of the genus *Cladophialophora* according to the disclosure include *Cladophialophora chaetospira, Cladophialophora arxii, Cladophialophora tortuosa, Cladophialophora floridana, Cladophialophora psammophila, Cladophialophora boppii, Cladophialophora hachijoensis, Cladophialophora carrionii, Cladophialophoratumbae,* and *Cladophialophora tumulicola.* From the viewpoint of more exhibiting the effect of the disclosure, *Cladophialophora chaetospira* is preferable.

As *Cladophialophora chaetospira, Cladophialophora chaetospira* SK 51 (hereinafter, also referred to as SK 51 or SK 51 isolate) deposited under the receipt number NITE ABP-03539 or a mutant isolate thereof is preferable. The SK 51 isolate has been deposited at the National Institute of Technology and Evaluation, Biotechnology Center Patent Microorganisms Depositary, having an address at 2-5-8 Kazusakamatari, Kisarazu-shi, Chiba, Japan (date of receipt: September 28, 2021).

The mutant isolate is more preferably a mutant isolate in which the action of promoting plant growth or flower bud formation under conditions usually not suitable for plant growth is equivalent to or more than the action of the SK 51 isolate. As a method of introducing a mutation, a method by treatment with a chemical substance such as a nitroso compound (for example, nitrosamine or nitrosoguanidine) or an alkylating agent (for example, EMS; ethyl methanesulfonate), ultraviolet irradiation, and radiation irradiation, but not limited to them. Whether or not the obtained mutant isolate exhibits the action equivalent to or more than that of the SK 51 isolate can be assayed by measuring the promoting action of the obtained mutant isolate on plant growth or flower bud formation and comparing the result with the measurement result of the action of the SK 51 isolate.

### (Other Fungi)

The culture soil for plants of the first embodiment may contain a fungus other than the fungus exemplified above as long as the effect of promoting plant growth or flower bud formation is not impaired. The types of other fungus are not particularly limited.

### (Content of Fungus)

In the culture soil for plants of the first embodiment, it is preferable that the fungus is prepared to be contained in an amount of 5% by mass to 10% by mass as a material containing the fungus from the viewpoint of more exhibiting the effect of the disclosure. In the disclosure, a material containing a fungus can be obtained by the following method. In detail, 10 mL of the preculture solution of the fungus (For example, 1 × 10⁵ hyphal fragments/ml to 1 × 10⁶ hyphal fragments/ml) is added to the sterilized plastic bag containing the sterilized culture material (For example, a mixture of 100 g of wheat bran, 100 g of rice bran, 300 g of humus and 340 mL of sterile water). Next, the mixture of the preculture solution of the fungus and the culture material is cultured in a chamber for 3 weeks to 4 weeks, whereby a material containing the fungus can be obtained.

The number of fungi in the culture soil for plants of the first embodiment may be, for example, 10³ hyphal fragments/g or more. Since plants and fungi are in a symbiotic relationship, the number of fungi in culture soil increases as the cultivation time of plants elapses. The number of fungi described above is preferably the number of fungi at the time point when the cultivation of the plant is started in the culture soil for plants of the first embodiment (For example, a time point at which a seed of the plant is seeded in the culture soil for plants, a time point at which a seedling at three-leaf stage separately grown is transplanted and planted in the culture soil for plants).

The number of fungi in the culture soil for plants of the first embodiment can be measured by plating fungi on a 50% by mass CMMY agar medium and then culturing at 23°C for 7 days.

### (Form of Fungus)

In the culture soil for plants of the first embodiment, the form of the fungus may be any form of the life cycle of the fungus. The form of the fungus may be, for example, a mycelium or a spore.

### <Soil>

### (Organic Cultivation Soil)

The organic cultivation soil according to the present disclosure refers to soil not containing agricultural chemicals and chemical fertilizers. Examples of the soil facing the organic cultivation soil include conventional cultivation soil (that is, inorganic cultivation soil), and the conventional cultivation soil refers to soil containing agricultural chemicals and/or chemical fertilizers.

### (Strongly Acidic Soil)

The strongly acidic soil according to the present disclosure is preferably soil having a pH of 3 or more and less than 5, and more preferably soil having a pH of 3 or more and less than 4, from the viewpoint of more exhibiting the effects of the present disclosure.

In the disclosure, the weakly acidic soil is soil having a pH of 5 or more and less than 7.

In the disclosure, the weakly acidic to neutral soil is soil having a pH of 5 or more and 7 or less, and this pH is usually a pH used as conventional cultivation soil.

In the disclosure, the pH of soil is measured by the following method. Specifically, the pH of soil can be confirmed by mixing soil and distilled water at a sample: solution ratio of 1: 2.5 (preferably 1: 5 when soil having a high organic matter content is used), stirring the mixture with a reciprocating shaker for 1 hour or more, and then measuring the pH of the suspension at 23°C ± 2 °C by a glass electrode method.

From the viewpoint of more exerting the effect of the present disclosure, it is preferable that the culture soil for plants of the first embodiment includes a fungus belonging to the genus *Cladophialophora*, and any one of strongly acidic organic cultivation soil, strongly acidic conventional cultivation soil, and weakly acidic to neutral organic cultivation soil, and the soil is used as the culture soil for plant growth promotion or the culture soil for plant flower bud formation promotion.

### <Other Components>

The culture soil for plants of the first embodiment may contain other components other than the soil and the fungus. Examples of other components include, but are not limited to, a solid medium (for example, an amino acid such as leucine, methionine, or phenylalanine, and sucrose) and a liquid medium (for example, water, sterile water, sterile distilled water, or physiological saline), a component for stably retaining the fungus according to the present disclosure in the culture soil (for example, a stabilizer, a tonicity agent), and a component for promoting the growth of the fungus according to the present disclosure (for example, malt extract medium (MEB), CM malt yeast medium (CMMY) (preferably a mixture of 8.5 g of CM agar, 15 g of agar, 10 g of malt extract, 1 g of yeast extract and 1 L of sterile water), wheat bran, rice bran, humus). The content of other components in the culture soil for plants of the first embodiment is not particularly limited as long as the effect of promoting the plant growth or the flower bud formation is not impaired.

### <Plant>

Since the culture soil for plants of the first embodiment can promote the plant growth or the flower bud formation under a condition that is usually not suitable for the growth of the plant, the plant is particularly excellent as the culture soil for plants of the fruits or vegetables. Alternatively, the kind of plant in the culture soil for plants may be a plant of the rose family (for example, strawberry, apricot, Japanese apricot, cherry, Japanese plum (plum), pear, Chinese pear, Japanese pear, loquat, peach, apple, prune, nectarine, karin, quince). In the culture soil for plants of the first embodiment, the kind of the plant is more preferably strawberry.

### «Culture Soil for Plants of Second Embodiment>>

Culture soil for plants according to a second embodiment of the present disclosure includes the fungus of *Exophiala* sp. SK 47 or a mutant isolate thereof, and strongly acidic organic cultivation soil or strongly acidic conventional cultivation soil.

The culture soil containing the fungus that is *Exophiala* sp. SK 47 or the mutant isolate thereof and the strongly acidic organic cultivation soil or the strongly acidic conventional cultivation soil is the culture soil that can promote the plant growth or the flower bud formation under conditions that are usually not suitable for the growth of plants.

Although the action of the culture soil for plants of the second embodiment is not clear, as in the first embodiment, the plant growth or the flower bud formation is promoted by cultivating the plant with the culture soil containing the fungus even under conditions such as organic cultivation soil, strongly acidic soil, or temperature or day length not suitable for the plant growth or the flower bud formation as a result of symbiosis between the fungus which is *Exophiala* sp. SK 47 or the mutant isolate thereof and the plant.

Note that the disclosure is not limited to the estimation mechanism at all.

### <Fungi>

### (The fungus which is Exophiala sp. SK 47 or the Mutant Isolate Thereof)

*Exophiala* sp. SK 47 (hereinafter, also referred to as SK 47 or SK 47 isolate) according to the disclosure has been deposited at the National Institute of Technology and Evaluation, Biotechnology Center Patent Microorganisms Depositary, having an address at 2-5-8 Kazusakamatari, Kisarazu-shi, Chiba, Japan, under the receipt number NITE ABP-03540 (date of receipt: September 28, 2021).

The mutant isolate of *Exophiala* sp. SK 47 is more preferably a mutant isolate in which the action of promoting plant growth or flower bud formation under conditions usually not suitable for plant growth is equivalent to or more than the action of the SK 47 isolate. Examples of a method of introducing a mutation into *Exophiala* sp. SK 47 include a method by treatment with a chemical substance such as nitroso compound (for example, nitrosamine, nitrosoguanidine) or alkylating agent (for example, EMS, ethyl methanesulfonate), ultraviolet irradiation, and radiation irradiation but are not limited to them. Whether or not the obtained mutant isolate exhibits the action equivalent to or more than that of the SK 47 isolate can be assayed by measuring the promoting action of the obtained mutant isolate on plant growth or flower bud formation and comparing the result with the measurement result of the action of the SK 47 isolate.

The description of (Other Fungi), (Content of Fungus), (Form of Fungus), (Organic Cultivation Soil), and (Strongly Acidic Soil) in the culture soil for plants of the second embodiment is the same as the description described in the item of «Culture Soil for Plants of First Embodiment>>, including definitions, examples, preferred embodiments, and the like.

From the viewpoint of more exerting the effect of the present disclosure, it is preferable that the culture soil for plants of the second embodiment includes a fungus which is *Exophiala* sp. SK 47 or the mutant isolate thereof, and the strongly acidic conventional cultivation soil, and this culture soil is used as the culture soil for plant growth promotion. Alternatively, from the viewpoint of more exhibiting the effect of the present disclosure, it is preferable that the culture soil for plants of the second embodiment includes a fungus which is *Exophiala* sp. SK 47 or the mutant isolate thereof, and the strongly acidic organic cultivation soil or the strongly acidic conventional cultivation soil, and the soil is used as the culture soil for promoting flower bud formation of a plant.

The description of <Other Components> and <Plant> in the culture soil for plants of the second embodiment is the same as the description described in the item of «Culture Soil for Plants of First Embodiment>>, including definitions, examples, preferred embodiments, and the like.

### «Cultivation Set>>

A cultivation set according to the present disclosure includes the culture soil for plants of the first embodiment or the culture soil for plants of the second embodiment, and the plant body. According to the present disclosure, the cultivation set capable of promoting plant growth or flower bud formation even under conditions that are usually not suitable for plant growth is obtained.

### <Culture Soil for Plants of First Embodiment or Culture Soil for Plants of Second Embodiment>

The description of the culture soil for plants of the first embodiment or the culture soil for plants of the second embodiment in the cultivation set according to the present disclosure is the same as the description described in the item of «Culture Soil for Plants of First Embodiment» or «Culture Soil for Plants of Second Embodiment>>, including definitions, examples, preferred aspects, and the like.

### <Plant Body>

In the cultivation set according to the present disclosure, a plant body may be, for example, a seed or a seedling of a plant. The seed of the plant may be, for example, a seed in which a radicle or a juvenile bud appears. The seedling of the plant may be, for example, a seedling in which only cotyledon appear, a seedling of three-leaf stage or more stage, a small seedling or a grandchild seedling (for example, runner seedling) grown from a parent isolate, or a clonal seedling grown by cutting.

With respect to the plant body in the cultivation set according to the disclosure, the description of the kind of the plant, including definitions, examples, preferred embodiments, and the like, is the same as the description of <Plant> described in the item of «Culture Soil for Plants of First Embodiment>>.

### «Plant Cultivation Method»

A method of cultivating a plant according to the disclosure includes a cultivation step of cultivating a plant using the culture soil for plants of the first embodiment or the culture soil for plants of the second embodiment. According to the method of cultivating a plant according to the disclosure, it is possible to promote the plant growth or flower bud formation even under a condition that is usually not suitable for the growth of the plant.

### <Cultivation Step>

### (Culture Soil for Plants)

The description of culture soil for plants in the method of cultivating a plant according to the disclosure is the same as the description described in the item of «Culture Soil for Plants of First Embodiment>> or «Culture Soil for Plants of Second Embodiment>>, including definitions, examples, preferred embodiments, and the like.

### (Plant)

The description of the types of plants in the plant cultivation method according to the disclosure, including definitions, examples, preferred embodiments, and the like, is the same as the description of <Plant> described in the item of «Culture Soil for Plants of First Embodiment>>.

### (Cultivation Conditions)

From the viewpoint that the effect of the disclosure is more exhibited, it is preferable that the cultivation step in the plant cultivation method according to the present disclosure includes cultivating a plant in which flower bud formation occurs under a low-temperature and short-day condition under a high-temperature and long-day condition.

Specifically, the plant in which flower bud formation occurs under the low-temperature and short-day condition is preferably a plant that forms flower buds regardless of the day length at a temperature of more than 5°C and 15°C or less (that is, low-temperature), forms flower buds only under the short-day condition at a temperature of more than 15°C and 25°C or less (that is, high-temperature), and does not form flower buds regardless of the day length at a temperature of more than 25°C. In the present disclosure, the short-day condition means that the day length is 0 hour or more and 12 hours or less.

Examples of plants in which flower bud formation occurs under low-temperature conditions include strawberry, broad bean, pea, broccoli, cauliflower, and the like. Examples of the plant in which flower bud formation occurs under short-day conditions include strawberry, cereals such as soybean and rice, and flowers and ornamental plants such as chrysanthemum and morning glory.

In the method of cultivating the plant according to the present disclosure, as the high-temperature condition, the temperature is preferably in a range of more than 15°C and 25°C or less, more preferably in a range of more than 20°C and 25°C or less, and still more preferably 23°C. In the plant cultivation method according to the disclosure, as long-day conditions, the day length is preferably within a range of more than 12 hours and 24 hours or less, more preferably within a range of more than 14 hours and 24 hours or less, and still more preferably 16 hours.

In the disclosure, the temperature is controlled by the following method. Specifically, the phytotron used for raising plants is maintained at a constant temperature, and the raising place is rotated every several days, so that the uniformity of the temperature can be secured.

### <Supply Step>

The plant cultivation method according to the disclosure may include a supply step. The supply step may be a step of supplying a fungus belonging to the genus *Cladophialophora* or a fungus which is *Exophiala* sp. SK 47 or a mutant isolate thereof to the soil.

### (Supply Method)

As a method of supplying fungi to soil, a method of mixing soil and fungi is preferable. When the fungus is supplied to the soil, <Other Components> described in the item of «Culture Soil for Plants of First Embodiment>> may also be supplied to the soil.

### (Supply Time)

The time when the fungus is supplied to the soil may be a time point when seeds of plants are seeded in the soil, a time point when seedlings of three-leaf stages are planted in the soil, or a time point when seedlings are planted in the soil. Alternatively, the supply may be performed by sowing seeds of plants, planting seedlings at the three-leaf stage, or planting seedlings in the soil mixed with fungi in advance. From the viewpoint of further achieving the effects of the present disclosure, fungi may be resupplied to the culture soil not only in the supply step but also in the cultivation step.

### (Supply Amount)

From the viewpoint of more achieving the effect of the present disclosure, the fungus to be supplied to the soil is preferably supplied so as to be contained in an amount of 5% by mass to 10% by mass as a material containing the fungus in the culture soil for plants. In the disclosure, a method of obtaining a material containing a fungus is as described in the item of «Culture Soil for Plants of First Embodiment>>.

The number of fungi supplied to the soil may be, for example, 10³ hyphal fragments/g or more in culture soil.

### «Seedling of Plant with Culture Soil»

The seedling of the plant with culture soil according to the disclosure is the culture soil for plants according to the disclosure and the seedling of the plant.

According to the disclosure, it is possible to obtain a plant seedling capable of promoting growth or flower bud formation even under conditions that are usually not suitable for plant growth.

A preferable aspect of the plant in the seedling of the plant with culture soil according to the disclosure is the same as the description of <Plant> described in the item of «Culture Soil for Plants of First Embodiment».

### EXAMPLES

Hereinafter, the disclosure will be described more specifically with reference to examples, but the disclosure is not limited to the following examples unless it goes beyond the gist of the disclosure. Unless otherwise specified, "parts" is on a mass basis. "%" is also based on mass.

### « Supply Step»

### <Preparation of Material Containing Fungus>

As fungi contained in the culture soil for plants, three kinds of dark-septate endophytes, *Exophiala* sp. SK 47 (hereinafter, also referred to as SK 47), *Exophiala pisciphila* SK 48 (hereinafter, also referred to as SK 48), and *Cladophialophora chaetospira* SK 51 (hereinafter, also referred to as SK 51) were prepared. *Exophiala* sp. SK 47 is a fungus deposited at the National Institute of Technology and Evaluation, Biotechnology Center Patent Microorganisms Depositary, having an address at 2-5-8 Kazusakamatari, Kisarazu-shi, Chiba, Japan, under the receipt number NITE ABP-03540. *Cladophialophora chaetospira* SK 51 is a fungus deposited at the National Institute of Technology and Evaluation, Biotechnology Center Patent Microorganisms Depositary, having an address at 2-5-8 Kazusakamatari, Kisarazu-shi, Chiba, Japan, under the receipt number NITE ABP-03539. These three fungi were each cultured in a 500 mL volume flask containing 250 mL of 2% by mass malt extract medium (MEB) under shaking conditions of 23°C and 120 rpm for 4 weeks. After the culture, three types of mycelia were respectively collected by filtering the culture solution, and the mycelia were washed with sterile distilled water until the liquid containing the collected mycelia became clear so that no substances derived from the MEB medium were brought in. Thereafter, the obtained mycelia and sterilized distilled water were mixed at a minimum speed for 1 minute using a mixer and a laminar flow in order to prevent contamination, thereby obtaining a preculture solution. The survival rate of each fungus in the preculture solution was measured by directly plating the preculture solution containing the mycelia on 50% by mass CMMY agar medium, and then culturing at 23°C for 7 days.

The mass production of each fungus was performed by the following method. Specifically, 10 mL of the preculture solution of each fungus (1 × 10⁶ hyphal fragments/ml) was added to the sterilized plastic bag containing the sterilized culture material (mixture of 100 g of wheat bran, 100 g of rice bran, 300 g of humus and 340 mL of sterile water). Then, the mixture of the preculture solution and the culture material of each fungus was cultured in a chamber for 3 weeks to 4 weeks to prepare a material containing each fungus.

### <Preparation of Soil>

As organic cultivation soil (the soil that is a sustainable agricultural model), organic soil (manufactured by SAKATA SEED CORPORATION) was prepared. As conventional cultivation soil (Also referred to as inorganic cultivation soil. That is, as soil which is a conventional agricultural method model), "Powerful soil for strawberries N50 for seedlings" (manufactured by Takara Industry Co., Ltd.) was prepared.

Since the prepared organic cultivation soil had a pH of 6.0 to 6.5, this was defined as "weakly acidic to neutral organic cultivation soil". On the other hand, "strongly acidic organic cultivation soil" having a pH of 3.8 to pH 4.3 was prepared by mixing an appropriate amount of peat moss (manufactured by TACHIKAWAHEIWANOUEN CO., LTD.) with the prepared organic cultivation soil.

The prepared conventional cultivation soil had a pH of 6.0 to 6.5, and was thus defined as "weakly acidic to neutral conventional cultivation soil". On the other hand, "strongly acidic conventional cultivation soil" having a pH of 3.8 to pH 4.3 was prepared by mixing an appropriate amount of peat moss (manufactured by TACHIKAWA HEIWA NOHEN CO., LTD.) with the prepared conventional cultivation soil.

The pH of the soil was measured by the following method. Specifically, soil and distilled water were mixed at a sample: solution ratio of 1: 2.5 (1: 5 in the case of using soil having a high organic matter content), and the mixture was stirred with a reciprocating shaker for 1 hour or more, and then the pH of the suspension was measured at 23°C ± 2°C by a glass electrode method.

All the above soils were sterilized by pressure 2 times at 121°C and 30 minutes.

### <Supply of Fungus to Soil>

Materials containing dark-septate endophytes of *Exophiala* sp. SK 47, *Exophiala pisciphila* SK 48, or *Cladophialophora chaetospira* SK 51 were mixed with the prepared weakly acidic to neutral organic cultivation soil, weakly acidic to neutral conventional cultivation soil, strongly acidic organic cultivation soil, and strongly acidic conventional cultivation soil so as to be 10% by mass in the culture soil, thereby preparing the culture soil for plants of the first embodiment (hereinafter, also referred to as first culture soil), the culture soil for plants of the second embodiment (hereinafter, also referred to as second culture soil), the culture soil as comparative examples, and the culture soil as reference examples. The weakly acidic to neutral conventional cultivation soil used in the reference examples is soil that can be said to be generally suitable for the growth of plants.

That is, culture soil under the following conditions was prepared.
(Comparative Example 1) Strongly acidic organic cultivation soil
(Example 1; Second Culture Soil) Strongly acidic organic cultivation soil and *Exophiala* sp. SK 47
(Comparative Example 2) Strongly acidic organic cultivation soil and *Exophiala pisciphila* SK 48
(Example 2; First Culture Soil) Strongly acidic organic cultivation soil and *Cladophialophora chaetospira* SK 51
(Comparative Example 3) Strongly acidic conventional cultivation soil
(Example 3; Second Culture Soil) Strongly acidic conventional cultivation soil and *Exophiala* sp. SK 47
(Comparative Example 4) Strongly acidic conventional cultivation soil and *Exophiala pisciphila* SK 48
(Example 4; First Culture Soil) Strongly acidic conventional cultivation soil and *Cladophialophora chaetospira* SK 51
(Comparative Example 5) Weakly acidic to neutral organic cultivation soil
(Comparative Example 6) Weakly acidic to neutral organic cultivation soil and *Exophiala* sp. SK 47
(Comparative Example 7) Weakly acidic to neutral organic cultivation soil and *Exophiala pisciphila* SK 48
(Example 5; First Culture Soil) Weakly acidic to neutral organic cultivation soil and *Cladophialophora chaetospira* SK 51
(Reference Example 1) Weakly acidic to neutral conventional cultivation soil
(Reference Example 2) Weakly acidic to neutral conventional cultivation soil and *Exophiala* sp. SK 47
(Reference Example 3) Weakly acidic to neutral conventional cultivation soil and *Exophiala pisciphila* SK 48
(Reference Example 4) Weakly acidic to neutral conventional cultivation soil and *Cladophialophora chaetospira* SK 51

### «Cultivation Step»

### <Preparation of Plant>

As the plant, everbearing strawberry plant (Wild Strawberry, produced by SAKATA SEED CORPORATION) was used. The surface of strawberry seeds was sterilized by the following method. Specifically, seeds were immersed in a 70% by mass ethanol solution for 1 minute and 30 seconds, and immersed in a sodium hypochlorite (1% by mass effective chlorine) solution for 1 minute. Next, the seeds were washed three times with sterilized distilled water, dried overnight, then placed on 1.5% by mass water agar medium, cultured at 23°C to germinate, and grown for 4 weeks until reaching the three-leaf stage.

### <Planting on Culture Soil for Plants>

Each of the prepared culture soils of Examples 1 to 5, Comparative Examples 1 to 7, and Reference Examples 1 to 4 was placed in a pot having a diameter of 6 cm, and strawberry at the three-leaf stage was planted in the culture soil.

### <Cultivation Conditions>

Strawberry was cultivated at a constant temperature of 23°C and a day length of 16 hours for 150 days after planting as a high-temperature and long-day condition. That is, in the cultivation step in this example, the low-temperature treatment was not performed.

Incidentally, the temperature and the day length are related to the flower bud formation of strawberry. Strawberry generally forms flower buds regardless of day length at a temperature higher than 5°C and 15°C or lower (i.e., low-temperature), flower buds only under short-day conditions at a temperature higher than 15°C and 25°C or lower (i.e., high-temperature), and no flower buds regardless of day length at a temperature higher than 25°C. Therefore, usually, the strawberry undergoes a flower bud promotion treatment using a low-temperature storage at 15°C, that is, a low-temperature treatment.

### «Evaluation»

### <Growth State of Strawberry>

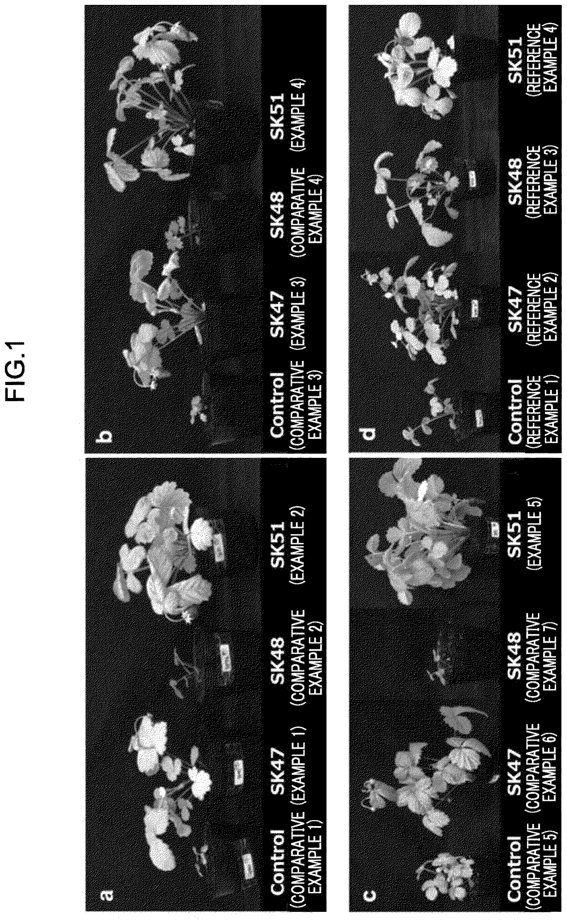

After cultivation in culture soil under various conditions, the growth state of the strawberry was visually observed. The results are shown in Fig. 1. In Fig. 1, a represents a growth state in strongly acidic organic cultivation soil, b represents a growth state in strongly acidic conventional cultivation soil, c represents a growth state in weakly acidic to neutral organic cultivation soil, and d represents a growth state in weakly acidic to neutral conventional cultivation soil. Control indicates culture soil of control group not containing fungus.

### <Dry Mass of Above-ground Part>

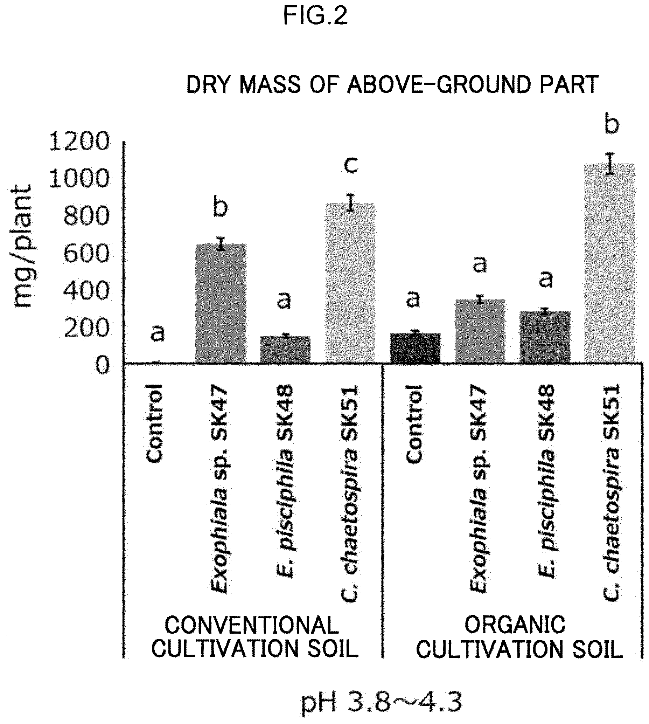

After cultivation in culture soil under various conditions, the strawberry plant body was taken out from each pot, and the strawberry was dried at 40°C for 72 hours, and then the mass of the above-ground part was measured. Statistical analysis was performed using SPSS version 20.0 (SPSS, IBM, Armonk, NY, United States), one-way analysis of variance (ANOVA) and Tukey's range test (p < 0.05). The results are shown in Fig. 2. In Fig. 2, in the bar graph under the same soil condition and the same pH condition, a and b, and a and c, indicate that there was a significant difference between them. The vertical axis represents the dry mass (mg) of the above-ground part of one strawberry plant body. Control indicates culture soil of control group not containing fungus.

That is, in the case of the strongly acidic conventional cultivation soil, when SK 47 or SK 51 was contained as the fungus in the culture soil, the growth of strawberry was promoted. In the case of strongly acidic organic cultivation soil, when SK 51 was contained as a fungus in the culture soil, the growth of strawberry was promoted.

### <Dry Mass of Root>

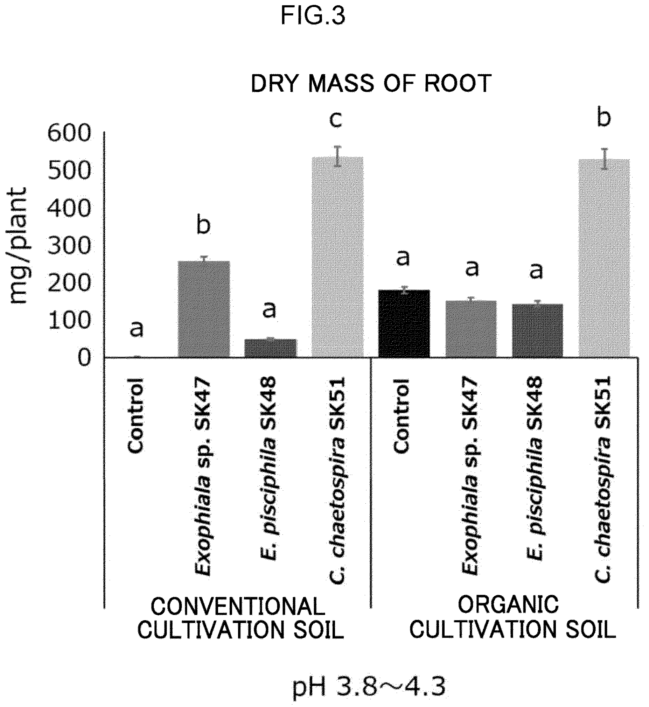

After cultivation in culture soil under various conditions, the strawberry plant body was taken out from each pot, and the strawberry was dried at 40°C for 72 hours, and then the mass of the root part was measured. The results are shown in Fig. 3. The method of statistical analysis and the symbols of a, b, and c are the same as the methods and symbols described in the item of <Dry Mass of Above-ground Part>. The vertical axis represents the dry mass (mg) of the root obtained from one strawberry plant body. Control indicates culture soil of control group not containing fungus.

That is, in the case of the strongly acidic conventional cultivation soil, when SK 47 or SK 51 was contained as the fungus in the culture soil, the growth of strawberry was promoted. In the case of strongly acidic organic cultivation soil, when SK 51 was contained as a fungus in the culture soil, the growth of strawberry was promoted.

### <Number of Fruits Formed>

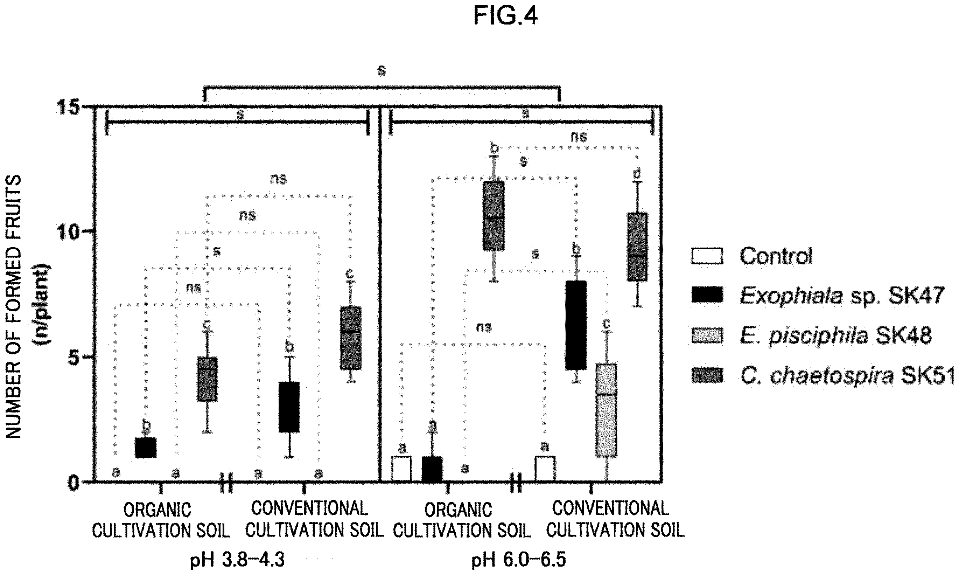

After cultivation in culture soil under various conditions, the number of fruits formed in strawberry was measured. The results are shown in Fig. 4. The method of statistical analysis and the symbols of a, b, and c are the same as the methods and symbols described in the item of <Dry Mass of Above-ground Part>. In the bar graph of the results of the weakly acidic to neutral conventional cultivation soil, a and d indicate that there was a significant difference therebetween. Note that s indicates that there is a significant difference, and ns indicates that there is no significant difference. The vertical axis represents the number of fruits (number) obtained from one strawberry plant body. Control indicates culture soil of control group not containing fungus.

That is, in the case of the strongly acidic conventional cultivation soil, when the SK 47 or the SK 51 was contained as the fungus in the culture soil, fruit formation of the strawberry was promoted. Also in the case of strongly acidic organic cultivation soil, when the SK 47 or the SK 51 was contained in culture soil as the fungus, fruit formation of the strawberry was promoted. In the case of weakly acidic to neutral organic cultivation soil, when the SK 51 as a fungus was contained in the culture soil, fruit formation of the strawberry was promoted. In the case of weakly acidic to neutral conventional cultivation soil (that is, inorganic cultivation soil) carried out as the reference examples, when the SK 47, the SK 48, or the SK 51 was contained as the fungus in the culture soil, fruit formation of the strawberry was promoted.

### <Days until Flower Bud Formation>

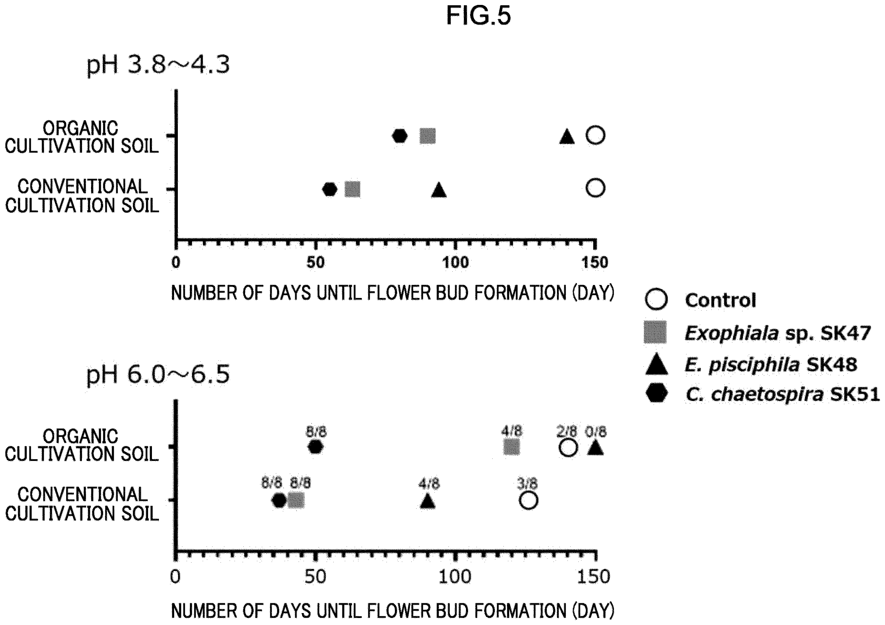

The number of days until flower bud formation of the strawberry was measured after cultivation in culture soil under various conditions. The results are shown in Fig. 5. In Fig. 5, the sample showing that the number of days until flower bud formation was 150 days indicates that the number of flower bud formation was 0 at the time point of 150 days. In the lower graph of Fig. 5 (pH 6.0 to 6.5), the number at the top of each plot indicates the number of individuals in which flower bud formation occurred in 8 individuals of strawberry. Control indicates culture soil of control group not containing fungus.

That is, in the case of the strongly acidic conventional cultivation soil, when the SK 47, the SK 48, or the SK 51 was contained as the fungus in the culture soil, the flower bud formation of the strawberry was promoted. In the case of strongly acidic organic cultivation soil, when the SK 47 or the SK 51 was contained as the fungus in the culture soil, the flower bud formation of the strawberry was promoted. In the case of weakly acidic to neutral organic cultivation soil, when the SK 51 as the fungus was contained in the culture soil, the flower bud formation of the strawberry was promoted. In the case of the weakly acidic to neutral conventional cultivation soil (that is, the inorganic cultivation soil) carried out as the reference examples, when the SK 47, the SK 48, or the SK 51 was contained in the culture soil as the fungus, the flower bud formation of the strawberry was promoted.

From the above results, under the high-temperature condition at a constant temperature of 23°C and the long-day condition with a day length of 16 hours in which the strawberry of the control group could not sufficiently form flower bud, the growth of the strawberry or the flower bud formation was promoted by inclusion of *Exophiala* sp. SK 47 or *Cladophialophora chaetospira* SK 51 in the culture soil. In particular, when *Cladophialophora chaetospira* SK 51 was contained in the culture soil, the promoting effect was remarkable.

That is, the culture soil for plants of the present disclosure promotes the growth or flower bud formation of strawberry even in the organic cultivation soil (that is, the soil which is sustainable agricultural model) or the soil which is strongly acidic (that is, the soil which is unsuitable model for cultivation) under the high-temperature and long-day condition which is usually unsuitable for the cultivation of strawberry. The fact that the strawberry can be harvested under the high-temperature and long-day condition means that the strawberry can be harvested throughout the year. Furthermore, since the fungus of the present disclosure exerts an effect by living in symbiosis with a plant, it is possible to promote growth or flower bud formation also on plants other than strawberry.

The disclosure of Japanese Patent Application No. 2021-190595, filed on November 24, 2021, is incorporated herein by reference in its entirety. All publications, patent applications, and technical standards mentioned in this specification are herein incorporated by reference to the same extent as if each individual publication, patent application, and technical standard were specifically and individually indicated to be incorporated by reference.

## Claims

1. A culture soil for plants, comprising:
a fungus of genus *Cladophialophora*; and
any one kind of soil of strongly acidic organic cultivation soil, strongly acidic conventional cultivation soil, or weakly acidic to neutral organic cultivation soil.

2. The culture soil for plants according to claim 1, wherein
the fungus of the genus *Cladophialophora* is *Cladophialophora chaetospira.*

3. Culture soil for plants, comprising:
a fungus of *Exophiala* sp. SK 47 or a mutant isolate thereof; and
strongly acidic organic cultivation soil or strongly acidic conventional cultivation soil.

4. The culture soil for plants according to any one of claims 1 to 3, wherein
the strong acidity is pH 3 or more and less than pH 5.

5. The culture soil for plants according to any one of claims 1 to 4, wherein
the plant is a fruit plant or a vegetable plant.

6. A cultivation set comprising:
the culture soil for plants according to any one of claims 1 to 5; and
a plant body.

7. A method of cultivating a plant, comprising:
a cultivation step of cultivating a plant using the culture soil for plants according to any one of claims 1 to 5.

8. The method of cultivating a plant according to claim 7, wherein
in the cultivation step, a plant in which flower bud formation occurs under a low-temperature and short-day condition is cultivated under a high-temperature and long-day condition.

9. The method of cultivating a plant according to claim 8, wherein
the high-temperature and long-day condition comprises a temperature in a range of more than 15°C and 25°C or less, and a day length in a range of more than 12 hours and 24 hours or less.

10. A seedling of a plant with culture soil, comprising:
the culture soil for plants according to any one of claims 1 to 5; and
a seedling of a plant.
